**Europäisches Patentamt**

**European Patent Office**

**Office européen des brevets**

(19)

(11) Veröffentlichungsnummer: **0 331 622 B1**

# EUROPÄISCHE PATENTSCHRIFT

(12)

(45) Veröffentlichungstag der Patentschrift:
**24.07.91 Patentblatt 91/30**

(51) Int. Cl.$^5$: **A61F 2/34**

(21) Anmeldenummer: **89810028.4**

(22) Anmeldetag: **12.01.89**

(54) **Halbkugelförmige, künstliche Hüftgelenkspfanne.**

(30) Priorität: **26.02.88 CH 718/88**

(43) Veröffentlichungstag der Anmeldung:
**06.09.89 Patentblatt 89/36**

(45) Bekanntmachung des Hinweises auf die
Patenterteilung:
**24.07.91 Patentblatt 91/30**

(84) Benannte Vertragsstaaten:
**AT DE FR GB IT**

(56) Entgegenhaltungen:
**EP-A- 0 226 762**
**EP-A- 0 265 712**
**DE-A- 3 205 526**
**DE-C- 3 341 723**
**DE-U- 8 623 855**
**FR-A- 2 595 241**

(73) Patentinhaber: **GEBRÜDER SULZER
AKTIENGESELLSCHAFT
Zürcherstrasse 9
CH-8401 Winterthur (CH)**

(72) Erfinder: **Dietschi, Carlo, Prof. Dr.-med.
Via nassa 46
CH-6900 Lugano (CH)**

## Beschreibung

Die Erfindung betrifft eine halbkugelförmige, künstliche Hüftgelenkspfanne mit einem Pfannenkörper aus Kunststoff, der aussen von einer Oberflächenstruktur aus Metall umhüllt ist, die entlang eines Meridians einen Längsschlitz aufweist.

Eine Hüftgelenkspfanne der vorstehend genannten Art ist bekannt aus der FR-A-24 16 004 ; diese bekannte Konstruktion besteht aus einer metallischen Aussenschale, in der ein Pfannenkörper aus Kunststoff verankert ist. Die Aussenschale ist dabei mit einer Oberflächenstruktur versehen ; weiterhin weist sie vier gleichmässig über den Umfang verteilte, in Meridianrichtung der Halbkugel verlaufende Schlitze auf. Diese haben die Aufgabe, ein gleichmässiges Aufspreizen der Aussenschale beim Einpressen des Pfannenkörpers zu ermöglichen, wodurch die Struktur auf der äusseren Oberfläche der Aussenschale zur Fixierung derselben in die Spongiosa des Beckenknochens eindringen soll.

In der Praxis hat sich nun gezeigt, dass diese Konstruktion in vielen Fällen eine zu grosse Steifigkeit besitzt, um den elastischen Bewegungen des Beckenknochens nachgeben zu können. Dies führt zu Relativ-"Verschiebungen" zwischen Knochen und Aussenschale, die auf der einen Seite Reizungen des Knochens und auf der anderen Seite Beschädigungen der äusseren Oberfläche der Aussenschale hervorrufen.

Es ist daher Aufgabe der Erfindung, eine aus Kunststoff bestehende, Hüftgelenkspfanne, deren dem Knochen zugewandten Oberfläche mit einer Metallstruktur versehen ist, zu schaffen, die in ihrer Elastizität der Elastizität des Beckenknochens nahe kommt. Diese Aufgabe wird nach der Erfindung dadurch gelöst, dass der Pfannenkörper mit einem mit dem Schlitz in der Oberflächenstruktur deckungsgleichen Längsschlitz versehen ist, der mindestens nahezu bis zum Pol der Halbkugel reicht, dass ferner die als im Kunststoff verankertes Metallgitter ausgebildete Oberflächenstruktur diametral gegenüber dem Längsschlitz mit einem am Beckenrand zu fixierenden Verankerungslappen versehen ist, und dass schliesslich die den Gelenkkopf einer zugehörigen Femurkopfprothese aufnehmende Pfannenschale aus zwei Hohlkugelflächen mit unterschiedlichen Radien zusammengesetzt ist, von denen der eine dem Radius des Gelenkkopfes entspricht, während der andere um die halbe Breite des Schlitzes an seinem äquatorialen Rand größer ist, wobei das Zentrum der grösseren Hohlkugelfläche auf dem Aequatordurchmesser zwischen Verankerungslappen und Längsschlitz gegenüber demjenigen der kleineren Hohlkugelfläche zum Längsschlitz hin versetzt ist.

Die erhöhte Elastizität gegenüber den bisherigen Konstruktionen ergibt sich daraus, dass der Pfannenkörper ebenfalls mit einem Meridianschlitz versehen ist und der Schlitz beider Elemente mindestens nahezu bis zum Pol der Halbkugel reicht. Das "Aufspalten" auch des Pfannenkörpers macht jedoch zusätzliche Massnahmen für eine einwandfreie Fixierung der Pfanne im Becken erforderlich ; weiterhin muss die Gefahr beachtet werden, dass der Gelenkkopf bei den elastischen Verformungen des Beckenknochens bzw. der Pfanne eingeklemmt werden kann. Die notwendige Fixierung am Beckenknochen wird durch den zusätzlichen Verankerungslappen gewährleistet, der mit Knochenschrauben am Becken befestigt und bei der Implantation so ausgerichtet wird, dass er in Richtung der Hauptbelastungen weist. Der Gefahr des Einklemmens des Gelenkkopfes ist durch den grösseren Durchmesser der Pfannenschale vorgebeugt.

Da die maximale elastische Zusammendrückbarkeit der Pfanne der Schlitzbreite am äquatorialen Rand entspricht, ist es vorteilhaft, wenn der Abstand beider Zentren der halben Schlitzbreite entspricht.

Anpassungen des Verankerungslappens an individuelle Beckenformen sind in gewissem Umfang möglich, wenn der Verankerungslappen intraoperativ bleibend verformbar ist ; dabei kann ergänzend zwischen Metallgitter und Verankerungslappen eine grabenförmige Rinne zur Aufnahme von Knochenspänen gebildet sein.

Falls erforderlich, ist zusätzlich eine Fixierung der relativ elastischen Pfanne im Becken möglich, wenn man beidseits des Längsschlitzes Durchtrittsöffnungen für Knochenschrauben vorsieht.

Im folgenden wird die Erfindung anhand eines Ausführungsbeispiels im Zusammenhang mit der Zeichnung näher erläutert.

Fig. 1    ist eine Aufsicht auf die neue Pfanne vom Aequator in Richtung auf den Pol gesehen ;
Fig. 2    gibt den Schnitt II-II von Fig. 1 wieder, während
Fig. 3    der Schnitt III-III von Fig. 1 ist.

Der die Pfannenschale 1 enthaltende Pfannenkörper 2 ist aussen in bekannter Weise mit einem nur schematisch dargestellten mehrlagigen Metallgitter 3 belegt, das mit mindestens einer Lage in den Kunststoff hineingepresst und auf diese Weise fixiert ist.

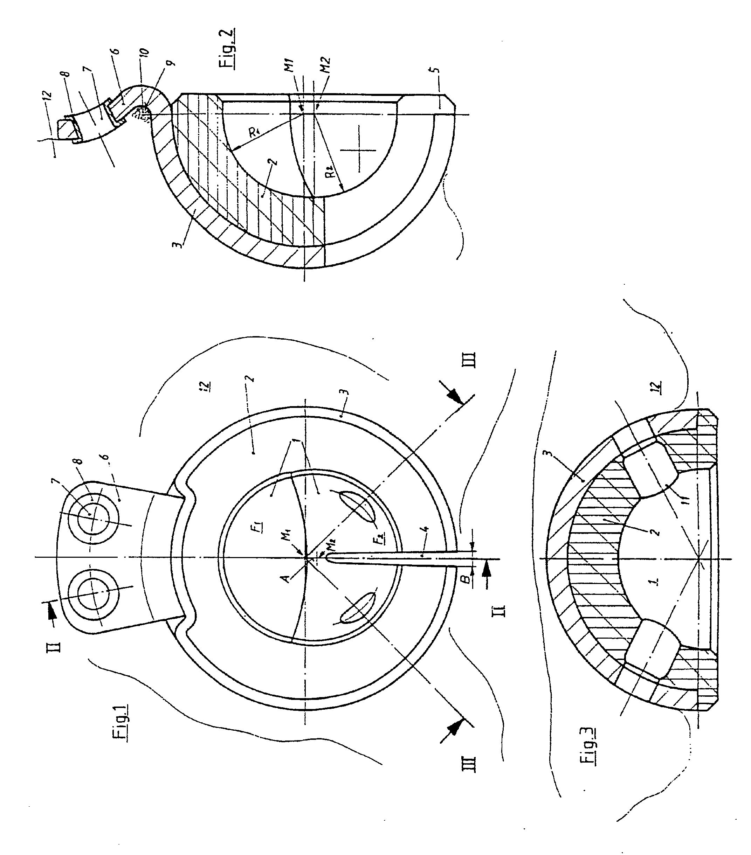

Pfannenkörper 2 und Metallgitter 3 sind entlang eines Meridians mit einem Schlitz 4 versehen, der sich nahezu bis zum Scheitel der Pfannenschale 1 erstreckt. Am äquatornahen Rand 5 (Fig. 2) beträgt die Schlitzbreite B ; sie hat beispielsweise einen Wert von etwa 3 mm.

Dem Schlitz 4 diametral gegenüberliegend ist am Metallgitter 3 ein Verankerungslappen 6 vorgesehen ;

2

dieser hat für den Durchtritt von nicht gezeigten Knochenschrauben zwei Bohrungen 7, die je mit einer Metallhülse 8 ausgekleidet sind. Die Hülsen 8 haben die Aufgabe, den Rand der Bohrungen 7 beim Anziehen der Knochenschrauben vor Beschädigungen zu schützen und darüberhinaus eine definierte Lage der Knochenschrauben zu gewährleisten. Zwischen der Halbkugelfläche der Pfanne und dem Verankerungslappen 6, der in gewissem Umfang intraoperativ bleibend verformbar ist, wird eine grabenförmige Rinne 9 gebildet, die bei der Implantation mit Knochenmaterial 10 gefüllt werden kann.

Die Pfannenschale 1 ist gebildet aus zwei Halbkugelflächen $F_1$ und $F_2$ mit unterschiedlichen Radien $R_1$ und $R_2$ und gegeneinander versetzten Mittelpunkten $M_1$ und $M_2$. Die Fläche $F_1$ bildet dabei den zum Verankerungslappen 6 gelegenen Teil der Pfannenschale 1 ; ihr Mittelpunkt ist $M_1$ und ihr Radius $R_1$ entspricht dem Radius des nicht gezeigten Gelenkkopfes der zugehörigen Femurkopfprothese. Der Radius $R_2$ der zweiten Schalenfläche $F_2$ ist grösser als der Radius $R_1$ ; sein Mittelpunkt $M_2$ ist gegenüber $M_1$ in der Aequatorebene der Pfanne in Richtung zum Schlitz 4 hin versetzt. Die Differenz der beiden Radien $R_1$ und $R_2$ und der Abstand A der Mittelpunkte $M_1$ und $M_2$ entsprechen je der halben Breite B/2 des Längsschlitzes 4. Beidseits des Schlitzes 4 sind Durchtrittsöffnungen 11 für Knochenschrauben zur weiteren Fixierung der Pfanne im Becken 12 vorgesehen.

Wie die schematisch dargestellten Beckenknochen 12 erkennen lassen, wird die neue Pfanne bei der Implantation so ausgerichtet, dass der Verankerungslappen 6 am Pfannendach anliegt und der Schlitz 1 im wesentlichen im und in Richtung des Einschnittes des Acetabulums verläuft. Bei einer solchen Anordnung stimmen Lage und Richtung des Verankerungslappens im wesentlichen mit der Richtung der Hauptbelastungskräfte überein, während die Pfannenabschnitte beiderseits des Längsschlitzes 4, die mit Knochenschrauben im Knochen zusätzlich fixiert sind, den elastichen Verformungen des Beckens folgen können, die im wesentlichen im Verengen und Erweitern des Schlitzes 4 bzw. des Einschnittes des Acetabulums bestehen. Der grosse "Hohlraum" der Pfannenschale 1 im Bereich der Hohlfläche $F_2$ verhindert, dass bei diesen Bewegungen der nicht gezeigte Gelenkkopf der Femurkopfprothese eingeklemmt werden kann.

## Patentansprüche

1. Halbkugelförmige, künstliche Hüftgelenkspfanne mit einem Pfannenkörper (2) aus Kunststoff, der aussen von einer Oberflächenstruktur aus Metall umhüllt ist, die entlang eines Meridians einen Längsschlitz aufweist, **dadurch gekennzeichnet,** dass der Pfannenkörper (2) mit einem mit dem Schlitz in der Oberflächenstruktur deckungsgleichen Längsschlitz (4) versehen ist, der mindestens nahezu bis zum Pol der Halbkugel reicht, dass ferner die als im Kunststoff verankertes Metallgitter (3) ausgebildete Oberflächenstruktur diametral gegenüber dem Längsschlitz (4) mit einem am Beckenrand zu fixierenden Verankerungslappen (6) versehen ist, und dass schliesslich die den Gelenkkopf einer zugehörigen Femurkopfprothese aufnehmende Pfannenschale (1) aus zwei Hohlkugelflächen ($F_1,F_2$) mit unterschiedlichen Radien ($R_1,R_2$) zusammengesetzt ist, von denen der eine dem Radius des Gelenkkopfes entspricht, während der andere um die halbe Breite (B/2) des Schlitzes (4) an seinem äquatorialen Rand (5) größer ist, wobei das Zentrum ($M_2$) der grösseren Hohlku-gelfläche ($F_2$) auf dem Äquatordurchmesser zwischen Verankerungslappen (6) und Längsschlitz (4) gegenüber demjenigen ($M_1$) der kleineren Hohlkugelfläche ($F_1$) zum Längsschlitz (4) hin versetzt ist.

2. Hüftgelenkspfanne nach Anspruch 1, **dadurch gekennzeichnet,** dass der Abstand (A) beider Zentren ($M_1,M_2$) der halben Schlitzbreite (B/2) entspricht.

3. Hüftgelenkspfanne nach Anspruch 1 oder 2, **dadurch gekennzeichnet,** dass der Verankerungslappen (6) intraoperativ bleibend verformbar ist.

4. Hüftgelenkspfanne nach einem der Ansprüche 1 bis 3, **dadurch gekennzeichnet,** dass beidseits des Längsschlitzes (4) Durchtrittsöffnungen (11) für Knochenschrauben vorgesehen sind.

5. Hüftgelenkspfanne nach einem der Ansprüche 1 bis 4, **dadurch gekennzeichnet,** dass zwischen Metallgitter (3) und Verankerungslappen (6) eine grabenförmige Rinne (9) zur Aufnahme von Knochenspänen gebildet ist.

## Claims

1. A hemispherical acetabular prosthesis having a plastics cup (2) enveloped externally in a metal surface structure formed with an elongate slot along a meridian, characterised in that the cup (2) is formed with an elongate slot (4) which coincides with the slot in the surface structure and which extends at least substantially as far as the pole of the hemisphere, the surface structure, in the form of metal mesh (3) anchored in the plastics, has diametrically opposite the slot (4) a fixing tab (6) fixable to the pelvis edge, and the cup shell (1) which receives the head of an associated femoral head prosthesis is combined from two hollow spherical surfaces ($F_1$, $F_2$) of different radii ($R_1$, $R_2$ respectively), one radius corresponding to the radius of the femoral head while the other is greater by half the width (B/2) of the slot (4) at its equatorial edge (5), the centre ($M_2$) of the larger hollow spherical surface ($F_2$) being offset along the equatorial diameter between the tab (6) and the slot (4) from the centre ($M_1$) of the smaller hollow spherical surface ($F_1$) towards the slot (4).

2. A prosthesis according to claim 1, characterised in that the between-centres distance (A) corresponds to half the slot width (B/2).

3. A prosthesis according to claim 1 or 2, characterised in that the tab (6) is permanently deformable intraoperatively.

4. A prosthesis according to any of claims 1-3, characterised in that apertures (11) through which bone screws can extend are present or either side of the slot (4).

5. A prosthesis according to any of claims 1-4, characterised in that a fossa-like groove (9) adapted to receive bone chips is present between the metal mesh (3) and the tab (6).

## Revendications

1. Cotyle artificiel de la hanche, en forme de demi-sphère, comportant un corps de cavité (2) en matière plastique qui est entouré extérieurement par une structure superficielle en métal qui présente une fente longitudinale le long d'un méridien, caractérisé en ce que le corps de cavité (2) est pourvu d'une fente longitudinale (4) superposable à la fente pratiquée dans la structure superficielle et qui s'étend au moins presque jusqu'au pôle de la demi-sphère, en ce qu'en outre la structure superficielle, se présentant sous la forme d'une grille métallique (3) ancrée dans la matière plastique, est pourvue, diamétralement par rapport à la fente longitudinale (4), d'une patte d'ancrage (6) à fixer sur le bord du bassin, et en ce qu'enfin la coque de cavité (1) recevant le condyle d'une prothèse de tête de fémur correspondante, est constituée de deux surfaces à concavité sphérique ($F_1$, $F_2$) de rayons ($R_1$, $R_2$) différents dont un correspond au rayon du condyle, tandis que l'autre est supérieur de la demi-largeur (B/2) de la fente (4), sur son bord équatorial (5), le centre ($M_2$) de la plus grande surface sphérique creuse ($F_2$) étant décalé, vers la fente longitudinale (4), sur le diamètre équatorial entre la patte d'ancrage (6) et la fente longitudinale (4), par rapport au centre ($M_1$) de la plus petite surface à concavité sphérique ($F_1$).

2. Cotyle de la hanche selon la revendication 1, caractérisée en ce que la distance (A) séparant deux centres ($M_1$, $M_2$) correspond à la moitié de la largeur de fente (B/ 2).

3. Cotyle de la hanche selon la revendication 1 ou 2, caractérisée en ce que la patte d'ancrage (6) est déformable de manière permanente au cours de l'opération.

4. Cotyle de la hanche selon l'une des revendications 1 à 3, caractérisée en ce qu'on prévoit de part et d'autre de la fente longitudinale (4), des ouvertures de passage (11) pour des vis d'os.

5. Cotyle de la hanche selon l'une des revendications 1 à 4, caractérisée en ce qu'une rigole en forme de tranchée (9) est formée entre la grille métallique (3) et la patte d'ancrage (6), pour recevoir des copeaux d'os.

Fig.1

Fig.2

Fig.3